# EUROPEAN PATENT SPECIFICATION

(11) **EP 2 964 192 B1**
(45) Date of publication and mention of the grant of the patent: **08.08.2018**
(21) Application number: 14730777.1
(22) Date of filing: 28.05.2014
(51) Int. Cl.: A61K 9/00, A61K 47/02, A61K 47/10, A61K 47/36, A61K 47/46, A61K 47/08, A61K 31/205

(54) **TRICHOLINE NASAL FORMULATION AND METHOD OF USE**
NASALE TRICHOLINFORMULIERUNG UND VERFAHREN ZUR VERWENDUNG
PRÉPARATION NASALE À BASE DE TRICHOLINE ET PROCÉDÉ D'UTILISATION

(30) Priority: 29.05.2013 US 201313904552
(43) Date of publication of application: 13.01.2016
(73) Proprietor: BRIU GmbH, 61462 Königstein (DE)
(72) Inventor: VOß, Christiane, 61476 Kronberg (DE); ROSE, Uwe-Bernd, 61462 Königstein (DE)
(74) Representative: Patent- und Rechtsanwälte Ullrich & Naumann
(86) International application number: PCT/EP2014/061119
(87) International publication number: WO 2014/191488

(56) References cited:
- US-A- 4 853 247
- US-A- 5 571 518
- US-A1- 2007 082 071
- LYDIA FERRARA ET AL: "Cytological Aspects on the Effects of a Nasal Spray Consisting of Standardized Extract of Citrus Lemon and Essential Oils in Allergic Rhinopathy", ISRN PHARMACEUTICS, vol. 40, no. 9, 1 January 2012 (2012-01-01), pages 39-6, XP055130470, DOI: 10.1007/978-1-4419-9533-9_12
- AMBROSE C S ET AL: "The safety and effectiveness of self-administration of intranasal live attenuated influenza vaccine in adults", VACCINE, ELSEVIER, AMSTERDAM, NL, vol. 31, no. 6, 30 January 2013 (2013-01-30), pages 857-860, XP002721189, ISSN: 0264-410X, DOI: 10.1016/J.VACCINE.2012.12.028 [retrieved on 2012-12-20]

## Description

### FIELD OF THE DISCLOSURE

The subject matter of the present disclosure generally relates to choline supplement products, and more particularly to a topically delivered tricholine citrate formulation for nasal administration.

### BACKGROUND OF THE DISCLOSURE

The use of choline products to relieve stress, mental pressure, anxiety, malaise, or "burn out" syndrome has been previously disclosed. Historically, these products were administered as injections, as oral administration is precluded by the metabolic profile of choline. Choline injectable products were previously marketed under the brand name Neurotropan. To be effective, the injections required regularly scheduled administration by a health care professional. Thus, patients' schedules had to be interrupted to allow for frequent doctor visits. Further, the injections caused multiple side effects as for example facial rash, feeling of pressure in head, tears, heavy breathing, tachycardy, vertigo, etc.
For this and other reasons, choline injection products ceased to be manufactured and available to patients and physicians.

The delivery of active ingredients in the form of nasal formulations has also been previously disclosed. However, preparation of a nasal formulation of choline presented various obstacles. First, an effective concentration of choline would need to be nasally administered. Second, a choline nasal product would need to have a commercially reasonable shelf-life. Third, the choline nasal product would need to be non-irritating. Fourth, the choline nasal product would need to be pleasing to the patient, i.e. the formulation should have a neutral or pleasant odor.

Tricholine citrate is a preferred choline source in a nasal formulation for various reasons. First, the tricholine salt provides a higher choline concentration on a weight/weight basis than other choline salts. The increased choline concentration allows for the delivery of comparatively larger quantity of choline per a similarly sized dose than other choline sources. Second, tricholine citrate is also readily soluble in water. Third, tricholine citrate is readily available from multiple commercial sources. However, the use of tricholine citrate in a nasal formulation presents several problems.

First, tricholine citrate has an odor that is considered repugnant, and therefore it would not readily appear viable as a candidate for delivery via nasal passageways, in which a substance's odor is of particular importance. Second, administration of a tricholine citrate aqueous solution may result in irritation to the nasal passageways and mucous membranes of a human, thereby creating discomfort for potential users. Third, an aqueous solution of tricholine citrate is unstable and will degrade relatively quickly, and result in an unacceptably short shelf-life and would not be considered commercially viable as a consumer product.

The subject matter of the present disclosure is directed to overcoming, or at least reducing the effects of, one or more of the problems set forth above.

### BRIEF SUMMARY OF THE INVENTION

The disclosed invention is a commercially-suitable choline nasal formulation that may be administered to humans. The composition includes an aqueous solution of tricholine citrate, a volatile oil and a buffer system. The buffer system serves to stabilize the compound while the volatile oil improves the odor of the composition. Optionally, a moisturizer is included in the composition to counter irritation from dryness that may result from administration of the composition to a user.

The disclosed tricholine nasal formulation has several advantages. First, the tricholine nasal formulation administered in a nasal spray eliminates the need for users to make frequent doctor visits for injections. Second, the tricholine nasal formulation is less irritating to the user's nasal passageways and mucous membranes, while still allowing for the effective administration of choline. The use of a buffer system reduces tricholine degradation and results in a commercially acceptable shelf-life for the product. Also, controlling the pH of the formulation within the disclosed range reduces nasal irritation. Other benefits of the disclosed subject matter may also be apparent to those in the art to which the disclosure pertains. Collectively, these attributes make the disclosed subject matter commercially viable as a product.

The details of one or more embodiments of the invention are set forth in the accompanying descriptions below. The foregoing summary is not intended to summarize each potential embodiment or every aspect of the disclosure.

### DETAILED DESCRIPTION OF THE DISCLOSURE

Disclosed is a tricholine nasal formulation, the composition of which overcomes several problems apparent in previously disclosed formulations and methods of delivery of choline products. The delivery of a choline product as a nasal spray is advantageous in that users can be given a nasal sprayer to use on their own, as opposed to requiring users to visit a doctor on a weekly or otherwise regular basis to receive injections. Therefore, cost and inconvenience for the user are reduced, making the tricholine nasal formulation better suited for the treatment of patients.

A basic embodiment composition includes an aqueous solution of tricholine citrate, a volatile oil, and buffer system in a concentration sufficient to stabilize the composition. Optionally an agent for increasing viscosity can be added. Various combinations of chemical compounds may serve as buffer systems and are known in the art. The volatile oil serves to at least partially, or ideally completely, improve or mask the odor of the overall composition when the composition is administered in an effective dosage to a user. Various volatile oils are suitable for this purpose and for use in conjunction with the disclosed subject matter. Optionally, such a volatile oil could be selected from among those approved for topical use as known in the art.

This basic embodiment may cause lubrication in the nasal cavity and mucous membranes of users. Additionally, it may be advantageous to include an moisturizer in the tricholine nasal formulation such that any irritation may be prevented or remedied. Optionally, such a moisturizer could be selected from among those already approved for topical use. An additional moisturizer may include a water thickener to aid in the retention of the composition at the site of application. Also the addition of natrosol 250 to increase viscosity is possible.

It may also be advantageous to include a preservative in the tricholine nasal formulation to enhance the longevity of the composition. For instance, phenoxyethanol or natrium-EDTA may be used for this purpose.

In a certain embodiment, a tricholine nasal formulation includes the following constituents: first, an aqueous solution of tricholine citrate in a concentration inclusive of 5 to 65 percent by weight; second, a buffer system designed to maintain the pH of the tricholine nasal formulation between 7.0 and 7.4 (a preferred buffer system includes sodium chloride, potassium chloride, disodium hydrogen phosphate and kalium-di-hydrogen-phosphate, in a concentration sufficient to maintain the pH of the composition at a value inclusive of 7.0 to 7.4); third, a moisturizer (hyaluronic acid or any methyl cellulose derivative), in a concentration sufficient to reduce the possibility of irritation caused by dryness when the tricholine nasal formulation is administered in an effective dose; natrasol 250 could also be added to increase viscosity; and lastly, a volatile oil (e.g., oil of citrus or oil of lavender) is used, in a concentration sufficient to substantially improve the odor of the composition of when the composition is administered in a tricholine nasal formulation.

In a specific demonstrated embodiment (Example 1), the above composition has the following relatively weighted values: tricholine citrate, preferably about 65 percent by weight, in a weight of about 1 g until 10 g, especially 1.6 g; optionally hyaluronic acid in a weight of 10 mg, sodium chloride in a weight of 80 mg, potassium chloride in a weight of 2 mg, disodium hydrogen phosphate in a weight of 11.5 mg, potassium-di-hydrogen phosphate in a weight of 2 mg, and oil of citrus in a weight of 50 mg.

### EXAMPLE 1

| | |
|---|---|
| tricholine citrate 65 % | 1.6 g |
| hyaluronic acid (optionally) | 10 mg |
| sodium EDTA (sodium2EDTA) | 0.05 g |
| Natrosol 250 | 0.1 g |
| sodium chloride | 80 mg |
| potassium chloride | 2 mg |
| disodium hydrogen phosphate | 11.5 mg |
| potassium-di-hydrogen phosphate | 2 mg |
| oil of citrus | 50 mg |
| distilled water | ad 10 g |

### EXAMPLE 2

| | |
|---|---|
| tricholine citrate 65 % | 1.28 g |
| sodium EDTA (sodium2EDTA) | 0.05 g |
| Natrosol 250 | 0.08 g |
| sodium chloride | 80 mg |
| potassium chloride | 2 mg |
| disodium hydrogen phosphate | 11.5 mg |
| potassium-di-hydrogen phosphate | 2 mg |
| oil of citrus | 50 mg |
| distilled water | ad 10 g |

As previously noted, the disclosed tricholine citrate composition is suitable for delivery by a nasal sprayer. There are various sprayers, many of which are used for delivering other medications or chemicals that would be expected to suffice for administration of the disclosed composition.

It is noted that an effective amount of an embodiment of the disclosed composition may be any amount sufficient to produce the desired effect on the user of the nasal spray. Alternatively, an effective amount may be defined as some amount of the composition that is found to be effective in a particularly large subset of users. The administration of an effective amount of the disclosed composition may require multiple sprays from a nasal sprayer, the number of which may depend on such factors as the desired dosage, the geometry or capability of the nasal sprayer, the user's typical affinity or lack thereof for the composition, or other factors.

## Claims

1. A tricholine citrate nasal spray composition, comprising:
an aqueous solution of tricholine citrate;
a volatile oil; and
a buffer system in a concentration sufficient to stabilize
the composition.

2. The composition of claim 1, wherein the tricholine citrate is in said aqueous solution in a concentration inclusive of 5 to 65 percent by weight.

3. The composition of claim 1, wherein said buffer system is in a concentration sufficient to adjust the pH of the composition to a value inclusive of 7.0 to 8.0.

4. The composition of claim 1, further comprising a moisturizer and/or an agent for increasing viscosity.

5. The composition of claim 4, wherein said moisturizer is in a concentration sufficient to reduce any irritation, for example caused by dryness, when that composition is administered in an effective amount as a nasal spray.

6. The composition of claim 4, wherein said moisturizer is hyaluronic acid.

7. The composition of claim 1, wherein said buffer system comprises sodium chloride, potassium chloride, disodium hydrogen phosphate and potassium-di-hydrogen phosphate.

8. The composition of claim 1, wherein said volatile oil is in a concentration sufficient to substantially improve the odor of said composition when said composition is administered in an effective amount as a nasal spray or wherein said volatile oil is oil of citrus.

9. The composition of claim 1, further comprising a preservative, wherein said preservative is Na EDTA.

10. The composition of claim 5, wherein:
said composition further includes a preservative;
said preservative is Na EDTA;
said moisturizer, optionally, is hyaluronic acid;
said buffer system comprises sodium chloride, potassium chloride, disodium hydrogen phosphate and potassium-di-hydrogen phosphate and is in a concentration sufficient to adjust the pH of the composition to a value inclusive of 7.0 to 8.0; and
said volatile oil is oil of citrus and is in a concentration sufficient to substantially improve the odor of said composition when said administering step is performed.

11. A tricholine citrate composition for use in medical treatment, wherein the tricholin citrate composition comprises an aqueous solution of tricholine citrate, a volatile oil and a buffer system in a concentration sufficient to stabilize the composition and wherein the use comprises the administration of the tricholine citrate composition as a nasal spray, and
wherein an effective amount of said tricholine citrate composition is administered when used as a nasal spray.

12. The tricholine citrate composition for use according to claim 11, wherein the tricholine citrate is in said aqueous solution in a concentration inclusive of 5 to 65 percent by weight.

13. The tricholine citrate composition for use according to claim 11, wherein said buffer system is in a concentration sufficient to adjust the pH of the composition to a value inclusive of 7.0 to 8.0.

14. The tricholine citrate composition for use according to claim 11, wherein said tricholine citrate composition further comprises a moisturizer, wherein said moisturizer is hyaluronic acid or wherein said moisturizer can be in a concentration sufficient to substantially reduce irritation caused by dryness, when the composition is administered in an effective amount as a nasal spray.

15. The tricholin citrate composition for use according to claim 11, wherein said buffer system comprises sodium chloride, potassium chloride, disodium hydrogen phosphate and potassium-di-hydrogen phosphate.

16. The tricholine citrate composition for use according to claim 11, wherein said volatile oil is in a concentration sufficient to substantially improve the odor of said composition when said administering step is performed.

17. The tricholine citrate composition for use according to claim 11, wherein said volatile oil is oil of citrus.

18. The tricholine citrate composition for use according to claim 11, further comprising a preservative, wherein said preservative is Na-EDTA.

19. The tricholine citrate composition for use according to claim 14, wherein:
said tricholine citrate composition further includes a preservative;
said preservative is Na-EDTA;
said moisturizer is hyaluronic acid;
said buffer system comprises sodium chloride, potassium chloride, disodium hydrogen phosphate and potassium-di-hydrogen phosphate and is in a concentration sufficient to adjust the pH of the composition to a value inclusive of 7.0 to 8.0; and
said volatile oil is oil of citrus and is in a concentration sufficient to substantially improve the odor of said composition when said administering step is performed.

## Patentansprüche

1. Eine Tricholincitrat-Nasenspray-Zusammensetzung, umfassend:
eine wässrige Lösung von Tricholincitrat;
ein flüchtiges Öl; und
ein Puffersystem in einer Konzentration, die zur Stabilisierung der Zusammensetzung ausreicht.

2. Die Zusammensetzung nach Anspruch 1, wobei das Tricholincitrat in der wässrigen Lösung in einer Konzentration von einschließlich 5 bis 65 Gew.-% vorliegt.

3. Die Zusammensetzung nach Anspruch 1, wobei das Puffersystem in einer Konzentration vorliegt, die ausreicht, um den pH-Wert der Zusammensetzung auf einen Wert von einschließlich 7,0 bis 8,0 einzustellen.

4. Die Zusammensetzung nach Anspruch 1, weiterhin umfassend einen Feuchtigkeitsspender und/oder ein Mittel zur Erhöhung der Viskosität.

5. Die Zusammensetzung nach Anspruch 4, wobei der Feuchtigkeitsspender in einer Konzentration vorliegt, die ausreicht, um jegliche Irritation zu reduzieren, beispielsweise verursacht durch Trockenheit, wenn diese Zusammensetzung in einer wirksamen Menge als Nasenspray verabreicht wird.

6. Die Zusammensetzung nach Anspruch 4, wobei der Feuchtigkeitsspender Hyaluronsäure ist.

7. Die Zusammensetzung nach Anspruch 1, wobei das Puffersystem Natriumchlorid, Kaliumchlorid, Dinatriumhydrogenphosphat und Kaliumdihydrogenphosphat umfasst.

8. Die Zusammensetzung nach Anspruch 1, wobei das flüchtige Öl in einer Konzentration vorliegt, die ausreicht, um den Geruch der Zusammensetzung wesentlich zu verbessern, wenn die Zusammensetzung in einer wirksamen Menge als Nasenspray verabreicht wird, oder wobei das flüchtige Öl Zitrusöl ist.

9. Die Zusammensetzung nach Anspruch 1, weiterhin umfassend ein Konservierungsmittel, wobei das Konservierungsmittel Na-EDTA ist.

10. Die Zusammensetzung nach Anspruch 5, wobei:
die Zusammensetzung weiterhin ein Konservierungsmittel enthält;
das Konservierungsmittel Na-EDTA ist;
der Feuchtigkeitsspender gegebenenfalls Hyaluronsäure ist;
das Puffersystem Natriumchlorid, Kaliumchlorid, Dinatriumhydrogenphosphat und Kaliumdihydrogenphosphat umfasst und in einer Konzentration vorliegt, die ausreicht, um den pH-Wert der Zusammensetzung auf einen Wert von einschließlich 7,0 bis 8,0 einzustellen; und
das flüchtige Öl Zitrusöl ist und in einer Konzentration vorliegt, die ausreicht, um den Geruch der Zusammensetzung wesentlich zu verbessern, wenn der Verabreichungsschritt durchgeführt wird.

11. Eine Tricholincitrat-Zusammensetzung zur Verwendung bei einer medizinischen Behandlung, wobei die Tricholincitrat-Zusammensetzung eine wässrige Lösung von Tricholincitrat, ein flüchtiges Öl und ein Puffersystem in einer Konzentration, die ausreicht, um die Zusammensetzung zu stabilisieren, umfasst und wobei die Verwendung die Verabreichung der Tricholincitrat-Zusammensetzung als ein Nasenspray umfasst, und
wobei eine wirksame Menge der Tricholincitrat-Zusammensetzung verabreicht wird, wenn sie als Nasenspray verwendet wird.

12. Die Tricholincitrat-Zusammensetzung zur Verwendung nach Anspruch 11, wobei das Tricholincitrat in der wässrigen Lösung in einer Konzentration von einschließlich 5 bis 65 Gew.-% vorliegt.

13. Die Tricholincitrat-Zusammensetzung zur Verwendung nach Anspruch 11, wobei das Puffersystem in einer Konzentration vorliegt, die ausreicht, um den pH-Wert der Zusammensetzung auf einen Wert von einschließlich 7,0 bis 8,0 einzustellen.

14. Die Tricholincitrat-Zusammensetzung zur Verwendung nach Anspruch 11, wobei die Tricholincitrat-Zusammensetzung weiterhin einen Feuchtigkeitsspender umfasst, wobei der Feuchtigkeitsspender Hyaluronsäure ist oder wobei der Feuchtigkeitsspender in einer Konzentration vorliegen kann, die ausreicht, um durch Trockenheit verursachte Irritation wesentlich zu reduzieren, wenn die Zusammensetzung in einer wirksamen Menge als Nasenspray verabreicht wird.

15. Die Tricholincitrat-Zusammensetzung zur Verwendung nach Anspruch 11, wobei das Puffersystem Natriumchlorid, Kaliumchlorid, Dinatriumhydrogenphosphat und Kaliumdihydrogenphosphat umfasst.

16. Die Tricholincitrat-Zusammensetzung zur Verwendung nach Anspruch 11, wobei das flüchtige Öl in einer Konzentration vorliegt, die ausreicht, um den Geruch der Zusammensetzung wesentlich zu verbessern, wenn der Verabreichungsschritt durchgeführt wird.

17. Die Tricholincitrat-Zusammensetzung zur Verwendung nach Anspruch 11, wobei das flüchtige Öl Zitrusöl ist.

18. Die Tricholincitrat-Zusammensetzung zur Verwendung nach Anspruch 11, weiterhin umfassend ein Konservierungsmittel, wobei das Konservierungsmittel Na-EDTA ist.

19. Die Tricholincitrat-Zusammensetzung zur Verwendung nach Anspruch 14, wobei:
die Tricholincitrat-Zusammensetzung weiterhin ein Konservierungsmittel enthält;
das Konservierungsmittel Na-EDTA ist;
der Feuchtigkeitsspender Hyaluronsäure ist;
das Puffersystem Natriumchlorid, Kaliumchlorid, Dinatriumhydrogenphosphat und Kaliumdihydrogenphosphat umfasst und in einer Konzentration vorliegt, die ausreicht, um den pH-Wert der Zusammensetzung auf einen Wert von einschließlich 7,0 bis 8,0 einzustellen; und
das flüchtige Öl Zitrusöl ist und in einer Konzentration vorliegt, die ausreicht, um den Geruch der Zusammensetzung wesentlich zu verbessern, wenn der Verabreichungsschritt durchgeführt wird.

## Revendications

1. Composition de pulvérisation nasale à base de citrate de tricholine, comprenant :
une solution aqueuse de citrate de tricholine ;
une huile volatile ; et
un système tampon en une concentration suffisante pour stabiliser la composition.

2. Composition selon la revendication 1, dans laquelle le citrate de tricholine est dans ladite solution aqueuse en une concentration de 5 à 65 pour cent en poids inclus.

3. Composition selon la revendication 1, dans laquelle ledit système tampon est en une concentration suffisante pour ajuster le pH de la composition à une valeur de 7,0 à 8,0 inclus.

4. Composition selon la revendication 1, comprenant en outre un hydratant et/ou un agent pour augmenter la viscosité.

5. Composition selon la revendication 4, dans laquelle ledit hydratant est en une concentration suffisante pour réduire toute irritation, par exemple causée par une sécheresse, lorsque cette composition est administrée en une quantité efficace en tant que pulvérisation nasale.

6. Composition selon la revendication 4, dans laquelle ledit hydratant est de l'acide hyaluronique.

7. Composition selon la revendication 1, dans laquelle ledit système tampon comprend du chlorure de sodium, du chlorure de potassium, de l'hydrogénophosphate de disodium et du di-hydrogénophosphate de potassium.

8. Composition selon la revendication 1, dans laquelle ladite huile volatile est en une concentration suffisante pour sensiblement améliorer l'odeur de ladite composition lorsque ladite composition est administrée en une quantité efficace en tant que pulvérisation nasale ou dans laquelle ladite huile volatile est de l'huile d'agrumes.

9. Composition selon la revendication 1, comprenant en outre un agent de conservation, dans laquelle ledit agent de conservation est de l'EDTA de Na.

10. Composition selon la revendication 5, dans laquelle :
ladite composition inclut en outre un agent de conservation ;
ledit agent de conservation est de l'EDTA de Na ;
ledit hydratant, facultativement, est de l'acide hyaluronique ;
ledit système tampon comprend du chlorure de sodium, du chlorure de potassium, de l'hydrogénophosphate de disodium et du di-hydrogénophosphate de potassium et est en une concentration suffisante pour ajuster le pH de la composition à une valeur de 7,0 à 8,0 inclus ; et
ladite huile volatile est de l'huile d'agrumes et est en une concentration suffisante pour sensiblement améliorer l'odeur de ladite composition lorsque ladite étape d'administration est réalisée.

11. Composition à base de citrate de tricholine pour utilisation dans un traitement médical, dans laquelle la composition à base de citrate de tricholine comprend une solution aqueuse de citrate de tricholine, une huile volatile et un système tampon en une concentration suffisante pour stabiliser la composition et dans laquelle l'utilisation comprend l'administration de la composition à base de citrate de tricholine en tant que pulvérisation nasale, et
dans laquelle une quantité efficace de ladite composition à base de citrate de tricholine est administrée lorsqu'elle est utilisée en tant que pulvérisation nasale.

12. Composition à base de citrate de tricholine pour utilisation selon la revendication 11, dans laquelle le citrate de tricholine est dans ladite solution aqueuse en une concentration de 5 à 65 pour cent en poids inclus.

13. Composition à base de citrate de tricholine pour utilisation selon la revendication 11, dans laquelle ledit système tampon est en une concentration suffisante pour ajuster le pH de la composition à une valeur de 7,0 à 8,0 inclus.

14. Composition à base de citrate de tricholine pour utilisation selon la revendication 11, dans laquelle ladite composition à base de citrate de tricholine comprend en outre un hydratant, dans laquelle ledit hydratant est de l'acide hyaluronique ou dans laquelle ledit hydratant peut être en une concentration suffisante pour sensiblement réduire une irritation causée par une sécheresse, lorsque la composition est administrée en une quantité efficace en tant que pulvérisation nasale.

15. Composition à base de citrate de tricholine pour utilisation selon la revendication 11, dans laquelle ledit système tampon comprend du chlorure de sodium, du chlorure de potassium, de l'hydrogénophosphate de disodium et du di-hydrogénophosphate de potassium.

16. Composition à base de citrate de tricholine pour utilisation selon la revendication 11, dans laquelle ladite huile volatile est en une concentration suffisante pour sensiblement améliorer l'odeur de ladite composition lorsque ladite étape d'administration est réalisée.

17. Composition à base de citrate de tricholine pour utilisation selon la revendication 11, dans laquelle ladite huile volatile est de l'huile d'agrumes.

18. Composition à base de citrate de tricholine pour utilisation selon la revendication 11, comprenant en outre un agent de conservation, dans laquelle ledit agent de conservation est de l'EDTA de Na.

19. Composition à base de citrate de tricholine pour utilisation selon la revendication 14, dans laquelle :
ladite composition à base de citrate de tricholine inclut en outre un agent de conservation ;
ledit agent de conservation est de l'EDTA de Na ;
ledit hydratant est de l'acide hyaluronique ;
ledit système tampon comprend du chlorure de sodium, du chlorure de potassium, de l'hydrogénophosphate de disodium et du di-hydrogénophosphate de potassium et est en une concentration suffisante pour ajuster le pH de la composition à une valeur de 7,0 à 8,0 inclus ; et
ladite huile volatile est de l'huile d'agrumes et est en une concentration suffisante pour sensiblement améliorer l'odeur de ladite composition lorsque ladite étape d'administration est réalisée.
